# EUROPEAN PATENT SPECIFICATION

(11) **EP 4 225 406 B1**
(45) Date of publication and mention of the grant of the patent: **12.08.2026**
(21) Application number: 21713680.3
(22) Date of filing: 25.03.2021
(51) Int. Cl.: A61M 5/315, A61M 5/24

(54) **DRUG DELIVERY DEVICE WITH ELECTRONIC SYSTEM**
ARZNEIMITTELABGABEVORRICHTUNG MIT ELEKTRONISCHEM SYSTEM
DISPOSITIF D'ADMINISTRATION DE MÉDICAMENTS AVEC SYSTÈME ÉLECTRONIQUE

(30) Priority: 27.03.2020 EP 20315066; 18.06.2020 EP 20315305; 23.07.2020 EP 20315357; 16.11.2020 EP 20315451
(43) Date of publication of application: 16.08.2023
(73) Proprietor: Sanofi, 75017 Paris (FR)
(72) Inventor: MORRIS, Anthony, Paul, Warwick Warwickshire CV34 4AB (GB); EARDLEY, Zoë, Georgina, Warwick Warwickshire CV34 4AB (GB); MARSH, William, Geoffrey, Arthur, Warwick Warwickshire CV34 4AB (GB); VEASEY, Robert, Warwick Warwickshire CV34 4AB (GB)
(74) Representative: Keil & Schaafhausen Patentanwälte PartGmbB
(86) International application number: PCT/EP2021/057669
(87) International publication number: WO 2021/191326

(56) References cited:
- WO-A1-2019/040313
- US-A1- 2016 015 903
- US-A1- 2019 269 858

## Description

The present invention is generally directed to an electronic system for a drug delivery device. The present invention further relates to a drug delivery device, which preferably comprises the electronic system.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is applicable for disposable and reusable devices.

For such devices the functionality of recording doses that are dialled and delivered from the pen may be of value to a wide variety of device users as a memory aid or to support detailed logging of dose history. Thus, drug delivery devices using electronics are becoming increasingly popular in the pharmaceutical industry as well as for users or patients. For example, a drug delivery device is known from EP 2 729 202 B1 comprising an electronically controlled capturing system for capturing data related to the amount of drug expelled from a reservoir by expelling means.

However, especially if the device is designed to be self-contained, that is to say without a connector for a connection to an electrical power source which is necessary to provide electrical power for the operation of the device, the management of the resources of a power supply integrated into the device is particularly important.

Unpublished EP 20315066.9 and EP 20315357.2 disclose advantageous embodiments of electronic systems for drug delivery devices with improved power management. Further, US 2016/015903 A1, WO 2019/040313 A1 and US 2019/269858 A1 describe drug delivery devices comprising electronic components for detecting operations of the drug delivery devices.

It is an object of the present disclosure to provide improvements for drug delivery devices comprising an electronic system or electronic systems for drug delivery devices.

This object is solved for example by the subject matter defined in the independent claim. Advantageous embodiments and refinements are subject to the dependent claims. However, it should be noted that the disclosure is not restricted to the subject matter defined in the appended claims. Rather, the disclosure may comprise improvements in addition or as an alternative to the ones defined in the independent claims as will become apparent from the following description.

One aspect of the disclosure relates to an electronic system for a drug delivery device comprising a use detection function. Another aspect of the disclosure relates to a drug delivery device comprising the electronic system. Accordingly, the features described herein with relation to the drug delivery device should be considered as being disclosed for the electronic system and vice versa.

The present disclosure provides advantageous embodiments relating to the integration of one or more mechanically activated electronic switches to initiate different device functions. The at least one switch may form or may be part of a use detection unit of the electronic system. Such a use detection unit may comprise the use of a rotationally activated electronic switch (rotational switch) to wake an electronic encoding module attached to an injection device and/or the use of an axially activated electronic switch (axial switch) to initiate pairing functionality of an encoding module, e.g. a rotary sensor, attached to an injection device, with another smart electronic device. A mechanically activated electronic switch may be or may form part of an electrical use detection unit operatively connected to an electronic control unit. The electrical use detection unit may be configured to generate a first signal which is indicative that the user has commenced or finished the relative movement between the first member and the button module. Thus, the present invention permits an injection device to maintain a low power state when energising of the encoding sensor(s) or pairing is not required, but to wake when either functionality is required. This is especially applicable to devices where a module rotates relative to an axially adjacent mechanism component during dose delivery but does not rotate relative to that component during dialing and/or to devices where the module moves axially relative to an adjacent mechanism component during the transition from a dialling to a dispensing state, or when the button module is pressed in a 0U dialled condition, i.e. a state at the completion of dose dispensing and prior to selecting a new dose.

According to one aspect of the present invention an electronic system comprises a dose setting and drive mechanism which is configured to perform a dose setting operation for setting a dose to be delivered by the drug delivery device and a dose delivery operation for delivering the set dose. The dose setting and drive mechanism comprises at least a first member which may be directly or indirectly operable by a user during the dose setting operation and/or the dose delivery operation. For example, the dose setting and drive mechanism may comprise one or more of the following components: a dial grip, a dial or display member (e.g. a number sleeve), a driver, a clutch, a piston rod, an inner and/or outer housing component. The dose setting and drive mechanism of the present invention may be based on the dose setting and drive mechanism disclosed in EP 2 890 435.

According to a further aspect of the present invention an electronic system comprises a button module comprising at least an electronic control unit, e.g. consisting of or comprising a PCBA, i.e. a PCB with the electronic components mounted, being configured to control an operation of the electronic system. The button module may be permanently or detachably attached to a trigger, a button or a dial grip, e.g. at or near the proximal end of the drug delivery device. The button module and/or the electronic control unit may have a distal surface facing towards the dose setting and drive mechanism, for example for providing an interface for mechanical interaction and/or electrical connection with further component parts of the system. As an example, the distal surface may comprise at least two, e.g. four, contact pads of the electronic control unit which may be selectively connected and dis-connected with electronic components, such as switching components.

In one embodiment, the electronic system has a first state and a second state. The first state and the second state may be different states of operation of the electronic system. The electronic control unit may have at least a first, preferably low power consumption, state and a second, preferably high power consumption, state. In the first state, the system may be in an idle state, where the system could not operate with the desired functionality assigned to the electronic system, e.g. use detection, motion detection, encoding, syncronisation and/or pairing. In other words, at least one function may be not activated in this first state. In the second state, the system may be ready to operate with the desired functionality, e.g. when the system is triggered to start an operation and/or when in the second state a dose setting operation and/or a dose delivery operation is being performed. The electronic system may have an increased electrical power consumption in the second state as compared to the first state. For example, in the second state, one or more electrical or electronic units of the electronic system may be switched to a state of higher power consumption, e.g. an on state, as compared to the first state, where the respective unit may be in a sleep state with low power consumption or an off state with no power consumption at all, e.g. because the connection to an electrical power supply is interrupted. For example, a communication unit and/or an encoding module. e.g. a rotary sensor, may be activated in this second state.

An encoding module or unit is typically suitable to detect a motion of a specific component part of the dose setting and drive mechanism and to generate signals indicative of the amount of motion of this component part. For example, the encoding module or unit may detect rotational movement of the first element, e.g. an encoder ring attached to a dial sleeve, during the dose setting operation and/or during the dose delivery operation. According to one aspect of the present disclosure, the encoding module comprises a rotary sensor for detection of a rotational movement. A rotary sensor may comprise a light source with a corresponding optical sensor, preferably two light sources with two corresponding optical sensors, for detecting a rotational movement of a component part having a pattern. As an alternative, a rotary sensor may use other detection techniques, e.g. the rotary sensor may comprise an electrical sliding contact, a mechanical switching arrangement and/or a magnetic sensor.

In a further aspect of the present disclosure, the button module and the dose setting and drive mechanism are configured such that the first member rotates relative to the button module during the dose delivery operation but does not rotate relative to the button module during the dose setting operation. In addition or as an alternative, the button module moves axially relative to the first member during the transition from the dose setting operation to the dose delivery operation, or when the button module is pressed in a 0U dialled condition. In other words, at least one relative movement of the button module with respect to the first member may occur in a predefined use situation of the drug delivery device and/or the electronic system.

According to an aspect of the present invention the electronic system further comprises at least two, e.g. four, contact pads of the electronic control unit, e.g. arranged at the distal surface, and at least one switch member with at least two corresponding switch contacts defining a first electrical state, e.g. an electrically open circuit, if one of the switch contacts is not connected to a corresponding contact pad of the button module and a second electrical state, e.g. an electrically closed circuit, if both contact pads of the button module are connected to the corresponding switch contacts.

According to a further aspect of the present disclosure, rotatation and/or axial movement of the button module relative to the first member may generate a, e.g. axial, movement component of the at least one switch member relative to the button module and the first member, thereby switching between the first and second electrical states. This may in turn trigger switching between the first low power consumption state and the second high power consumption state. In other words, an independent aspect of the invention is directed to at least one mechanically activated electronic switch, e.g. two switches, to initiate different system or device functions, e.g. the use of an, e.g. rotationally, activated electronic switch may wake an electronic encoding module attached to an injection device and/or the use of an, e.g. axially, activated electronic switch may initiate a pairing functionality of the button module with another smart electronic device. The at least one switch may be considered as an electrical use detection unit or as a part thereof, indicating, especially to other components of the electrical system, the use of the drug delivery device or a function or part thereof.

In the electronic system, the at least two contact pads of the electronic control unit may comprise at least a first contact pad and a second contact pad. Further, the at least two corresponding switch contacts of the switch member may comprise at least a first elastically deformable switch contact, e.g. permanently, abutting the first contact pad and a second elastically deformable switch contact, e.g. arranged axially offset to the first elastically deformable switch contact, spaced from the second contact pad in an unbiased condition or idle state or position. With this exemplary configuration a simple and yet reliable mechanically activated electronic switch may be used to initiate one or more different system or device functions. In a further example, two switch members are provided to allow initiating two or more different system or device functions. For example, the electronic system may comprise at least four contact pads and two switch members, wherein each switch member comprises at least a first elastically deformable switch contact permanently abutting one of the contact pads and a second elastically deformable switch contact, e.g. arranged axially offset to the first elastically deformable switch contact, spaced from one of the further contact pads in an unbiased condition or idle state or position.

The first elastically deformable switch contact does not need to be permanently abutting the contact pad for either switch to function. For example, in an embodiment the rotary switch may, in some tolerance conditions, not maintain contact between the first deformable switch contact and the contact pad. The design is such that the first deformable contact biases the second out of contact with the PCB contact pad, breaking the circuit in the unbiased condition. However, the axial switch design preferably does maintain contact between the first deformable switch contact and the contact pad in all tolerance conditions, following assembly. One reason for the rotary switch not following the same approach is to minimise the losses induced in the mechanism (and therefore increase in the dispense force that the user must apply to deliver a dose). If the first switch contact had to remain in abutment with the contact pad in all tolerance cases then it would be required to deform more as the rocker is deflected radially inwards by the ratchet interface with the encoder ring. This increased deformation results in an increased reaction force that must be overcome by the encoder ring (and therefore mechanism).

According to an aspect of the present disclosure, an axially activated electronic switch may initiate a pairing functionality of the button module, e.g. attached to an injection device, with another smart electronic device. For example, the axial switch initiates pairing or a manual syncronisation of data between the electronic module and companion smartphone. In an embodiment of such an axially activated electronic switch, the switch member may comprise a switch chassis constraining a switch contact strip, e.g. a single switch contact strip, comprising the elastically deformable switch contacts. For example, the switch contact strip is substantially U-shaped with two legs, each forming one of the elastically deformable switch contacts with the switch contact strip being arranged such that the legs are axially offset to each other. The switch member may be interposed between a module chassis of the button module and the electronic control unit. An axially extending protrusion, e.g. a pin, of the switch chassis may extend towards the first member, such that when the button module moves axially towards the first member, e.g. during the transition from the dose setting operation to the dose delivery operation, or when the button module is pressed in a 0U dialled condition, one of the switch contacts is brought into abutment with one of the contact pads. In other words, the axial movement of the button module relative to the first member may be used to close an electrical contact which may in turn trigger switching between the first low power consumption state and the second high power consumption state.

According to a further aspect of the present disclosure, a rotationally activated electronic switch may wake a motion detection unit or an electronic encoding module, attached to an injection device. In an embodiment of such a rotationally activated electronic switch, the switch member may comprise a rocker constraining a switch contact pressing, e.g. a single switch contact pressing, comprising the elastically deformable switch contacts. The switch contact pressing may comprise two contact arms, the tips of which, in the assembled orientation, are axially offset to each other and form elastically deformable switch contacts. In an exemplary embodiment, the switch member is interposed between the module chassis of the button module and the electronic control unit with a radially extending protrusion of the rocker extending towards the first member, such that when the button module rotates relative to the first member, e.g. during the dose delivery operation, a radial motion of the protrusion of the rocker results in an axial motion at the switch contacts and one of the switch contacts is brought into abutment with one of the contact pads. The rocker may be pivotably guided in the button module. For example, an axle of the rocker may be clipped into pockets in the module chassis, such that radial motion of a tip of the rocker results in axial motion at the switch contacts. In other words, the rotational movement of the button module relative to the first member may be used to close an electrical contact which may in turn trigger switching between the first low power consumption state and the second high power consumption state.

According to an exemplary embodiment of the electronic system, the first member is a dial member, e.g. a part of a dial sleeve assembly, a rotatable number sleeve, or a member axially and/or rotationally locked thereto. In more detail, the first member may be a dial sleeve or a member axially and/or rotationally locked thereto which first member is axially displaceable relative to a housing of the dose setting and drive mechanism, e.g. along a helical path, at least in the dose delivery operation. This first member is preferably rotatable relative to a housing of the dose setting and drive mechanism, e.g. along a helical path, at least in the dose setting operation. The button module is preferably axially displaceable relative to the first member and rotationally constrained to the first member at least in the dose setting operation. Further, the button module is preferably rotationally constrained to the housing in a dose dispensing operation. The first member may be or may comprise an encoder ring suitable for detecting rotational movement. Such an encoder ring may be an integral part or may be attached to said dial member, e.g. a rotatable number sleeve. An encoder ring may have an, e.g. radial, ratchet profile for interaction with the radially extending protrusion of the rocker and/or an, e.g proximally facing, ring-shaped running face for interaction with the axially extending protrusion of the switch chassis.

In this example, as the axial force to close such a switch acts on the, e.g. proximal, surface of the encoder ring during dispense, therefore there is a torque loss which acts to increase the dispense force of the injection device. The proximal surface of the encoder ring may be arranged as a continuous ring to provide a smooth running face and minimise variation in dispense force. The moving contact occurring between two moulded polymer parts allows a material combination to be selected that maintains a low coefficient of friction at this interface. Preferably, at least a portion of the first member, e.g. the running face of the encoder ring and/or the ratchet profile of the encoder ring, and at least the respective contact portion of the switch member, e.g the pin of the switch chassis and/or the radial protrusion of the rocker, are made of a plastic material, e.g. a polymer material with a low friction coefficient. This may further improve performance compared to a metal contact sliding on a polymer surface.

For example, the encoder ring may further comprise a pattern provided at least on its outer surface which can be detected by the rotary sensor. According to one aspect, the rotary sensor comprises a primary sensor and secondary sensor which are configured to target specially adapted regions at the proximal end of the dial sleeve, e.g. at the encoder ring. In this example, the primary sensor and the secondary sensor may be light reflective sensors. Therefore, the specially adapted proximal regions of the dial sleeve or the encoder ring may be divided into at least one reflective area and at least one non-reflective (or absorbent) area. The rotary sensor may be an optical sensor emitting light from an LED whose light is reflected by the reflective region(s) of the encoder ring and the sensor detects the reflected light. The sensor then converts the detected light to an electrical output. The encoding or motion sensing unit may comprise one or more of such optical rotary sensor(s), for example two optical rotary sensors located circumferentially spaced about the encoder ring.

In an example of the present disclosure, the encoder ring may have three functions, namely interacting with the axial switch, e.g. during the transition from the dose setting operation to the dose delivery operation or when the button module is pressed in a 0U dialled condition, interacting with the rotational switch, e.g. during the dose delivery operation, and interacting with the rotary sensor, e.g. during the dose delivery operation. In addition, or as an alternative, it may be envisaged that the rotary sensor interacts with the encoder ring during dose setting such that a different mechanical arrangement may allow the sensor to be used for dial encoding.

The electronic system may further comprise an encoding or motion sensing unit which is in a sleeping mode in the first low power consumption state and which is activated in the second high power consumption state, and/or a communication unit for communicating with another device, which communication unit is in a sleeping mode in the first low power consumption state and which is activated in the second high power consumption state. In an exemplary embodiment of the present disclosure, the electronic system may comprise an encoding or motion sensing unit and a communication unit wherein both units may be independently activated or in a sleeping mode. Thus, there may be more than two power consumption states, namely a state where both units are deactivated or in a sleeping mode, a state where only the encoding or motion sensing unit is activated, a state where only the communication unit is activated and a state where both units are activated. The power consumption of the electronic system may be different for each of these four states. Nevertheless, only a first low power consumption state and a second high power consumption state are discussed herein for simplification reasons.

In an embodiment, the electronic system may be suitable to collect or measure dose data, e.g. corresponding to the set dose or the dispensed dose, using the encoding or motion sensing unit. Such dose data may be collected only in the second state of the system. In one embodiment, the encoding or motion sensing unit, when it is active, may be operable to gather motion data or measurement data relating to the movement of e.g. a dial member, a driver and/or a piston rod. The electronic control unit may be configured to convert this data into dose data, e.g. characteristic for the size of the dose which has been set or has been delivered in the respective operation. The encoding or motion sensing unit may be designed as described in unpublished EP 20315066.9 and EP 20315357.2.

The communication unit may comprise a wireless communication interface for communicating with another device, wherein the electronic system is configured such that it is switched from the first state into the second state by the electronic control unit in response to the first signal, thereby inducing the communication unit to initiate a manual syncronsation and/or a pairing with another device.

The electronic control unit may, in response to reception of the first signal issue a command, e.g. a signal, to another unit of the electronic system such that this unit is switched on or rendered operational. This unit may be the communication unit for communicating with another device, e.g. a wireless communications interface for communicating with another device via a wireless network such as Wi-Fi or Bluetooth^{®}, or even an interface for a wired communications link, such as a socket for receiving a Universal Series Bus (USB), mini-USB or micro-USB connector. Preferably, the electronic system comprises an RF, WiFi and/or Bluetooth unit as the communication unit. The communication unit may be provided as a communication interface between the system or the drug delivery device and the exterior, such as other electronic devices, e.g. mobile phones, personal computers, laptops and so on. For example, dose data may be transmitted by the communication unit to the external device. The dose data may be used for a dose log or dose history established in the external device.

In one embodiment, the communication unit comprises a wireless communication interface for communicating with another device, wherein the electronic system is configured such that it is switched from the first state into the second state by the electronic control unit in response to the first signal of the at least one switch of the use detection unit, thereby inducing the communication unit to initiate a manual syncronisation and/or a pairing with another device or to initiate a mode for amending the settings of the electronic system.

According to one aspect of the present invention an electronic system comprises a dose setting and drive mechanism, an electrical power supply, e.g. a rechargeable or non-rechargeable battery, an electronic control unit, an electrical use detection unit and an encoding or motion sensing unit and/or a communication unit for communicating with another device.

In one embodiment, the device or the electronic system comprises an electronic control unit, e.g. comprising a microprocessor or microcontroller. The electronic control unit may be configured to control operation of the drug delivery device or the electronic system. The electronic control unit may be arranged on a conductor carrier and electrically conductively connected with conductors on the conductor carrier. The conductor carrier may be a circuit board such as a printed circuit board. The conductor carrier may be retained in the interior of the user interface member of the system or the device. The power supply may be arranged in the interior of the electronic system such as in the interior of the user interface member.

According to one aspect of the present disclosure, the electronic system is applicable to limit the battery capacity requirement of an injection device, where it is advantageous to be able to have the device in a low power state when any electronic functionality is not required. This can be achieved by mechanical switches which are activated by relative motion between the electronic button module and adjacent components as required, e.g. the above exemplarily mentioned encoder ring as part of the dial sleeve assembly.

According to one aspect of the present disclosure, the functionality of a manual synchronization is to be initiated on pressing the button module, when the device is at 0U dialled. When the button module is pressed, in any device state, the button module is translated, e.g. together with a clutch, distally relative to the dial sleeve assembly. The nominal axial travel may be limited, e.g. to less than 3 mm, for example to between 1.5 mm and 2.0 mm, travel of the button module relative to the dial sleeve (and the encoder ring), further relative axial motion is limited. The axial switch of one embodiment of the use detection unit is mounted in the underside of the button module and utilises the relative axial displacement between button module and dial sleeve assembly to trigger. The duration for which the button module is held in a depressed state may be used to allow multiple different functionalities to be initiated by the same switch, e.g. manual synchronisation for a short duration press and release, or pairing for a longer duration press and release.

According to one further aspect of the present disclosure, the functionality of e.g. encoding may be required to be initiated only when the device is dispensing. For example, in the device disclosed in EP 2 890 435, during dose setting the dial sleeve assembly, e.g. consisting of a dial sleeve and the encoder ring, and the button module extend (transalate) helically from the device. Therefore, there is no relative rotation between the button module and the dial sleeve assembly during dose setting. To initiate dose delivery the button module, e.g. and a clutch, are translated distally relative to the device housing. After the clutch has translated a predefined distance, e.g. less than 2.0 mm, for example nominally 1.20 mm, the clutch disengages from the dial sleeve and the delivery mechanism enters the dispensing (dose delivery) mode. In this dispensing mode the dial sleeve assembly retracts along the helical path into the device, whereas the button module does not rotate and only retracts with axial motion, until the 0U stop is engaged and dispense is complete. Thereby there is relative rotation of the button module with respect to the dial sleeve assembly during dispense. In the exemplary embodiment of a rotational switch of the use detection unit, this rotational switch may be mounted in the underside of the button module and utilises the relative rotation between the button module and dial sleeve assembly to trigger.

In this exemplary application to the device disclosed in EP 2 890 435, the axial switch will also be triggered when the button module is pressed as part of a dispensing event. However, with the embodiment described the relative order of the rotational and axial switch state changes cannot be guaranteed. It is possible that the axial switch will not be triggered before the point of clutch disengagement, e.g. 1.2mm button module translation, so some rotation of the dial sleeve assembly could occur prior to the axial switch state change. Use of the rotational switch to initiate e.g. an optical encoding system ensures that the delivered dose is accurately recorded, irrespective of the axial position of the button module. Without the requirement to trigger prior to clutch disengagement, the maximum deflection of the axial switch contacts and therefore the forces, stresses and package space of this axial switch can be minimised.

According to a further aspect of the present disclosure, the use detection unit comprises the axial switch and the rotational switch wherein the electronic control unit is adapted to switch the encoding or motion sensing unit into its low power consumption state in response to a signal that the axial switch is switched from its first electrical state, e.g. an electrically open circuit, into its second electrical state, e.g. an electrically closed circuit. In more detail, when the user releases the button module at the end of dispense (or midway through a dispense event) the button module and clutch translate proximally relative to the device, e.g. under a clutch spring force. The axial switch state will change during this motion but the rotational switch state will not. The change of state of the axial switch following a dispense event, provides information to the controller (electronic control unit) that the user has released the button module. Without this information, an increased delay period would be required prior to a readout of the dispensed dose being displayed, since the system must wait to check for no further rotational switch signals to determine if the dose is complete. This would have a negative implication on battery life and user experience. Thus, although the use detection unit may comprise only the axial switch or only the rotational switch, a combination of the axial switch and the rotational switch provides additional benefits exceeding the possibility of triggering two different functions with two different switches.

According to a further aspect of the present disclosure, the use detection unit comprises the axial switch wherein the rotational switch and the electronic control unit is adapted to generate a failure indicating signal, e.g. a signal which may be indicated and/or transmitted by means of the communication unit, if a difference in rotational positon is detected by the the encoding or motion sensing unit without detection of a state change of the rotational switch and/or if a rotational switch state change is received with no corresponding axial switch state change (however, detecting failure of the axial switch using the rotary switch might require reconfiguration of the switch points previously described; the axial switch would have to be adjusted to ensure it always closed before the axial clutch disengagement that allows dispense to start). In other words, the two switches may be used to check for correct functionality of each other and inform the user / disable the button module if there are any errors. For example, the system can be configured such that a single encoder position reading is taken whenever the axial switch is released. Gray code caching (e.g. as described in unpublished EP20315305.1 **)** enables the injection device to store the rotational position of the dial sleeve assembly at the end of previous use, and this can be compared to the new dial sleeve assembly position. If a difference in rotational positon is detected without detection of a state change of the rotational switch, the user can be alerted to an error and potential failure of the rotational switch. In the embodiment using the device disclosed in EP 2 890 435 two optical encoder sensors may be used. The Gray code caching resolution with this system is limited to 4 discrete sensor states, such that there is a 1 in 4 chance that a change in rotational position would not be detected if this mitigation was used in isolation. Inversely, if a rotational switch state change is received with no corresponding axial switch state change this may indicate a failure of the axial switch.

This can also be configured to prevent any further operation of the module independently of communicating to an external device - i.e. force permanent shutdown of that particular electronic module to prevent further use since it may not be operating reliably and there is consequently a risk of reporting incorrect dispensed dose values.

The present invention is applicable for devices which are manually driven, e.g. by a user applying a force to an injection button, for devices which are driven by a spring or the like and for devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting.

The present invention further pertains to a drug delivery device comprising the electronic system as described above. The drug delivery device may comprise a container receptacle which is releasably attached to the dose setting and drive mechanism. As an alternative, the container receptacle may be permanently attached to the dose setting and drive mechanism. The container receptacle is adapted to receive a container, e.g. a cartridge, containing a medicament.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (anti-diabetic drugs) or 86 (oncology drugs), and Merck Index, 15th edition. Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(w-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin. Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

The terms "axial", "radial", or "circumferential" as used herein may be used with respect to a main longitudinal axis of the device, the cartridge, the housing or the cartridge holder, e.g. the axis which extends through the proximal and distal ends of the cartridge, the cartridge holder or the drug delivery device.

Non-limiting, exemplary embodiments of the invention will now be described with reference to the accompanying drawings, in which:
- Figure 1: shows an embodiment of a drug delivery device;
- Figure 2a: shows a perspective view of an axial switch sub-assembly according to an embodiment of the present invention;
- Figure 2b: shows a perspective view of the axial switch sub-assembly during assembly into a module chassis;
- Figure 2c: shows a sectional view of the axial switch sub-assembly during assembly into a module chassis;
- Figure 2d: shows a sectional view of the axial switch sub-assembly during a further assembly step;
- Figure 2e: shows a sectional view of the axial switch sub-assembly and a PCB after assembly into a module chassis;
- Figure 2f: shows a sectional view of the axial switch during use;
- Figure 2g: shows a perspective view of the axial switch during use;
- Figure 2h: shows the contact pad locations on the underside of the PCB;
- Figure 3a: shows a perspective view of a rocker sub-assembly according to an embodiment of the present invention;
- Figure 3b: shows a sectional view of the rocker sub-assembly during assembly into a module chassis;
- Figure 3c: shows a bottom view of the rocker sub-assembly during assembly;
- Figure 3d: shows a sectional view of the rocker sub-assembly and a PCB after assembly into a module chassis;
- Figure 3e: shows a top view of the rocker sub-assembly;
- Figure 3f: shows a sectional view of the rocker in a first use position;
- Figure 3g: shows a sectional view of the rocker in a second use position; and
- Figure 4: illustrates schematically an embodiment of an electronic system for a drug delivery device.

In the figures, identical elements, identically acting elements or elements of the same kind may be provided with the same reference numerals.

In the following, some embodiments will be described with reference to an insulin injection device. The present disclosure is however not limited to such application and may equally well be deployed with injection devices that are configured to eject other medicaments or drug delivery devices in general, preferably pen-type devices and/or injection devices.

Embodiments are provided in relation to injection devices, in particular to variable dose injection devices, which record and/or track data on doses delivered thereby. These data may include the size of the selected dose and/or the size of the actually delivered dose, the time and date of administration, the duration of the administration and the like. Features described herein include the arrangement of sensing elements and power management techniques (e.g. to facilitate small batteries and/or to enable efficient power usage).

Certain embodiments in this document are illustrated with respect to the injection device disclosed in EP 2 890 435 where an injection button and grip (dose setting member or dose setter) are combined. The injection button may provide the user interface member for initiating and/or performing a dose delivery operation of the drug delivery device. The grip or knob may provide the user interface member for initiating and/or performing a dose setting operation. Both devices are of the dial extension type, i.e. their length increases during dose setting. Other injection devices with the same kinematical behaviour of the dial extension and button during dose setting and dose expelling operational mode are known as, for example, the Kwikpen^{®} device marketed by Eli Lilly and the Novopen^{®} 4 device marketed by Novo Nordisk. An application of the general principles to these devices therefore appears straightforward and further explanations will be omitted. However, the general principles of the present disclosure are not limited to that kinematical behaviour. Certain other embodiments may be conceived for application to Sanofi's SoloSTAR^{®} injection device where there are separate injection button and grip components / dose setting members. Thus, there may be two separate user interface members, one for the dose setting operation and one for the dose delivery operation.

"Distal" is used herein to specify directions, ends or surfaces which are arranged or are to be arranged to face or point towards a dispensing end of the drug delivery device or components thereof and/or point away from, are to be arranged to face away from or face away from the proximal end. On the other hand, "proximal" is used to specify directions, ends or surfaces which are arranged or are to be arranged to face away from or point away from the dispensing end and/or from the distal end of the drug delivery device or components thereof. The distal end may be the end closest to the dispensing and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be the needle end where a needle unit is or is to be mounted to the device, for example.

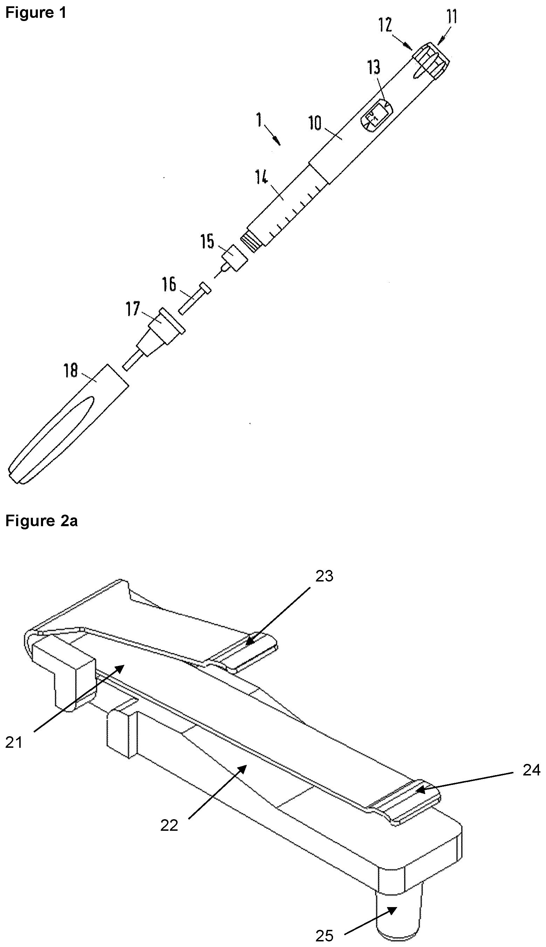

Figure 1 is an exploded view of a medicament delivery device or drug delivery device. In this example, the medicament delivery device is an injection device 1, e.g. a pen-type injector, such an injection pen disclosed in EP 2 890 435.

The injection device 1 of Figure 1 is an injection pen that comprises a housing 10 and contains a container 14, e.g. an insulin container, or a receptacle for such a container. The container may contain a drug. A needle 15 can be affixed to the container or the receptacle. The container may be a cartridge and the receptacle may be a cartridge holder. The needle is protected by an inner needle cap 16 and either an outer needle cap 17 or another cap 18. An insulin dose to be ejected from injection device 1 can be set, programmed, or 'dialled in' by turning a dosage knob or dial grip 12, and a currently programmed or set dose is then displayed via dosage window 13, for instance in multiples of units. The indicia displayed in the window may be provided on a number sleeve or dial sleeve. For example, where the injection device 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1.

The dosage window 13 may be in the form of an aperture in the housing 10, which permits a user to view a limited portion of a dial sleeve assembly 20 that is configured to move when the dial grip 12 is turned, to provide a visual indication of a currently set dose. The dial grip 12 is rotated on a helical path with respect to the housing 10 when setting a dose.

In this example, the dial grip 12 includes one or more formations to facilitate attachment of a data collection device. Especially, the dial grip 12 may be arranged to attach a button module 11 onto the dial grip 12. As an alternative, the dial grip may comprise such a button module of an electronic system.

The injection device 1 may be configured so that turning the dial grip 12 causes a mechanical click sound to provide acoustic feedback to a user. In this embodiment, the dial grip 12 also acts as an injection button. When needle 15 is stuck into a skin portion of a patient, and then dial grip 12 and/or the attached button module 11 is pushed in an axial direction, the insulin dose displayed in dosage display window 13 will be ejected from injection device 1. When the needle 15 of injection device 1 remains for a certain time in the skin portion after the dial grip 12 is pushed, the dose is injected into the patient's body. Ejection of the insulin dose may also cause a mechanical click sound, which may be different from the sounds produced when rotating the dial grip 12 during dialing of the dose.

In this embodiment, during delivery of the insulin dose, the dial grip 12 is returned to its initial position in an axial movement, without rotation, while the dial sleeve assembly 20 is rotated to return to its initial position, e.g. to display a dose of zero units. Figure 1 shows the injection device 1 in this 0U dialled condition. As noted already, the disclosure is not restricted to insulin but should encompass all drugs in the drug container 14, especially liquid drugs or drug formulations.

Injection device 1 may be used for several injection processes until either the insulin container 14 is empty or the expiration date of the medicament in the injection device 1 (e.g. 28 days after the first use) is reached. In the case of a resuable device, it is possible to replace the insulin container.

Furthermore, before using injection device 1 for the first time, it may be necessary to perform a so-called "prime shot" to remove air from insulin container 14 and needle 15, for instance by selecting two units of insulin and pressing dial grip 12 while holding injection device 1 with the needle 15 upwards. For simplicity of presentation, in the following, it will be assumed that the ejected amounts substantially correspond to the injected doses, so that, for instance the amount of medicament ejected from the injection device 1 is equal to the dose received by the user. Nevertheless, differences (e.g. losses) between the ejected amounts and the injected doses may need to be taken into account.

As explained above, the dial grip 12 also functions as an injection button so that the same component is used for dialling/setting the dose and dispensing/delivering the dose. As an alternative (not shown), a separate injection button may be used which is axially displaceable, at least a limited distance, relative to the dial grip 12 to effect or trigger dose dispensing.

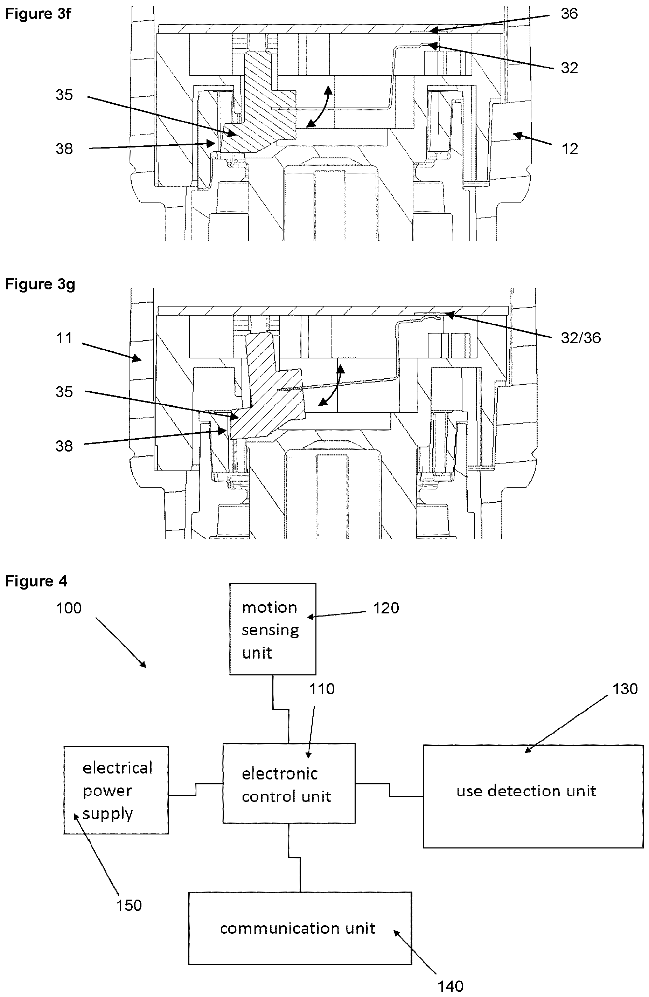

In the following, an electronic system 100 according to the invention will be described with respect to exemplary embodiments and with reference to Figure 4. The electronic system 100 comprises a dose setting and drive mechanism which may be part of an injection device 1 as depicted in Figure 1 and an electrical power supply 150, e.g. a rechargeable or non-rechargeable battery, as shown in Figure 4. The electronic system 100 further comprises an electronic control unit 110, e.g. comprising or consisting or being part of a PCBA, configured to control an operation of the electronic system 100 which has a first state and a second state, wherein the electronic system 100 has an increased electrical power consumption in the second state as compared to the first state. The electronic system 100 further comprises an encoding and motion sensing unit 120, e.g. a rotary sensor, and an electrical use detector unit 130 which is operatively connected to the electronic control unit 110 and which is configured to generate at least a first signal which is indicative that the user performs an operation. An example of such an operation is that the user of the injection device and/or the electronic system enters a manual synchronization or pairing mode of the electronic system 100 and/or that a user starts dose dispensing. The electronic system 100 is configured such that it is switched from the first state into the second state by the electronic control unit 110 in response to said first signal. The electronic system further comprises a communication unit 140 for communicating with another device. When the communication unit 140 is active to perform the manual synchronization or pairing mode, the electronic system 100 is in its second state.

Although not explicitly depicted, the electronic system 100 may comprise a, preferably permanent and/or non-volatile, storage or memory unit, which may store data related to the operation of the drug delivery device such as dose history data, for example.

Unless specifically disclosed otherwise in the following, the electronic system 100 may have the functions and may be arranged and/or designed as described in unpublished EP 20315066.9 and EP 20315357.2.

The present invention comprises alternatives for generating the first signal by means of an electrical use detector unit 130. In more detail, the embodiments are based on detecting an axial movement of a first member of the dose setting and drive mechanism with respect to the button module 11.

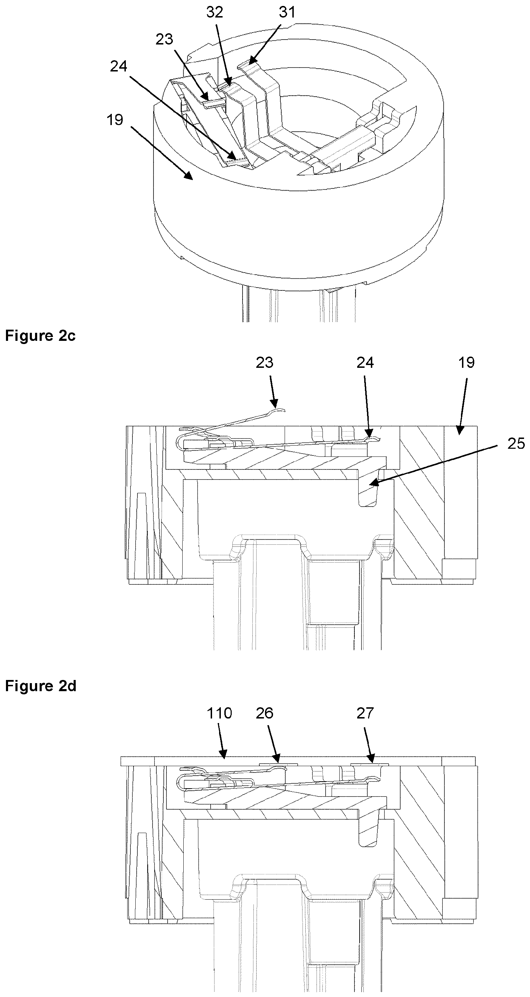

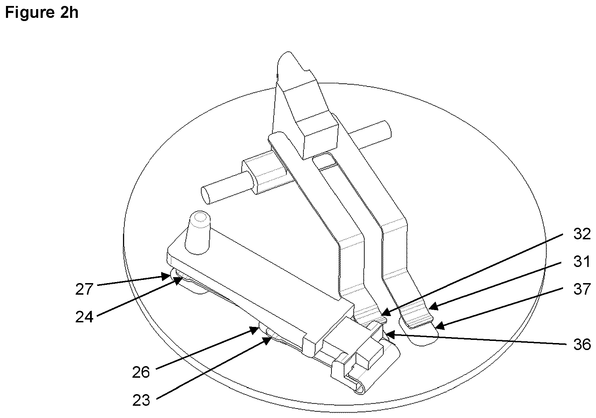

A first embodiment is depicted in Figures 2a to 2g. This embodiment is directed to an axial switch forming part of a use detection unit 130 of the electronic system 100. The use detection unit 130 is received within the button module 11. The button module 11 may be housed within the dial grip 12 or may be a separate unit attached to the dial grip 12. In the embodiment depicted in Figures 2a to 2g, the dial grip 12 has an, e.g. substantially cylindrical, outer shape with a profile allowing gripping and rotating the dial grip 12. The button module 11 is mainly received within the dial grip 12, wherein the dial grip 12 may form an outer housing of the button module 11. The dial grip 12 further houses a module chassis 19 fitted into the dial grip 12 during assembly of the device.

The dose setting and drive mechanism of the exemplary embodiment depicted in Figures 2a to 2g comprises at least a dial sleeve assembly in the form of a ring, e.g. an encoder ring 20, which may be permanently attached to a dial sleeve or number sleeve of the drug delivery device. As an alternative, the encoder ring 20 may be a unitary part of a dial sleeve assembly or number sleeve of the drug delivery device. In an embodiment, the dial sleeve assembly and, thus, the encoder ring 20 rotate and move axially on a helical path together with the button module 11 during dose setting. Further, the dial sleeve assembly and, thus, the encoder ring 20 rotate and move axially on a helical path in an opposite direction during dose delivery, wherein the button module 11 only moves axially such that a relative rotation occurs between the encoder ring 20 and the button module 11. Further, it may be possible to perform a relative axial movement, e.g. a limited axial movement, between the encoder ring 20 and the button module 11, for example for coupling and/or de-coupling the button module 11 or the encoder ring 20 from further components of the dose setting and drive mechanism.

As shown in Figure 2a, a potential embodiment of the axial switch comprises a single switch contact strip that is manufactured from stainless steel or beryllium copper etc. sheet or strip. The switch contact strip 21 is assembled onto a switch chassis 22 and comprises two elastically deformable arms each having a switch contact 23, 24 at its respective free end. The switch chassis 22 constrains the switch contact strip 21 from translation and positions the switch contacts 23, 24 in the correct position for assembly into the button module sub-assembly. As shown in Figure 2b, the axial switch sub-assembly is initially positioned into the module chassis 19 of the button module sub-assembly. A pin 25 on the switch chassis 22 component protrudes axially through a hole in the module chassis 19.

As shown in Figure 2d, the electronic control unit PCBA 110 is assembled onto the module chassis 19 and compresses the axial switch sub-assembly into the final assembly position.

The PCB has two exposed metallic pads 26, 27 on the distal surface. In this state the proximal surface of the switch contact 23 is in contact with one of the exposed pads 26 and is connected electronically to the PCB. The compression of the switch contact 23 may provide, e.g. approximately 0.2N, of pre-load, which ensures the switch chassis 22 is biased distally in the module chassis 19. The other switch contact 24 is in clearance to the PCB and the second pad 27 so that it is in a first electrical state (for example electrically open circuit in the depicted embodiment).

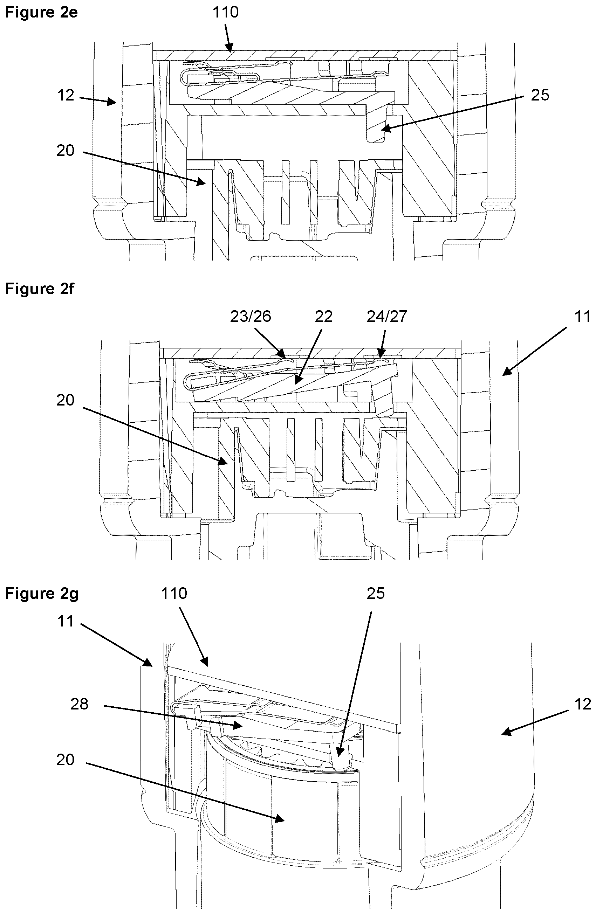

In the dialling state, when the button module 11 is assembled to the rest of the mechanism as shown in Figures 2e to 2g, there is axial clearance between the pin 25 and the encoder ring 20 which forms part of the dial sleeve assembly. As shown in Figures 2f and 2g, when the button module 11 is pressed it is displaced axially with respect to the dial sleeve assembly, ie. the encoder ring 20. Firstly the clearance between the switch chassis pin 23 and the encoder ring 20 is closed, followed by the switch chassis 22 being lifted to deflect the switch contact 24 proximally and close the clearance between the switch contact 24 and the second contact pad 27 on the PCB. Once this clearance has been closed, the electrical state of the switch is changed (for example to electrically closed in the depicted embodiment). This sequence operates in reverse when the button module 11 is released.

Thus, opening or closing the electrical contact between a switch contact 23 or 24 with a respective pad 26, 27 of the PCB may be used to wake up the electronic system or may trigger transition into a sleeping mode or other mode with comparably lower power consumption. Thus, the axial switch 23, 24, 26, 27 may be or form part of the electrical use detection unit 130.

The axial force to close the switch acts on the proximal surface of the encoder ring 20 during dispense, therefore there is a torque loss which acts to increase the dispense force of the injection device. The proximal surface of the encoder ring 20 is arranged as a continuous ring to provide a smooth running face 28 and minimise variation in dispense force. The moving contact occurring between two moulded polymer parts allows a material combination to be selected that maintains a low coefficient of friction at this interface, thus, performance is better than a metal contact sliding on a polymer surface.

The switch has been arranged such that the electrical change of state of the switch occurs prior to full displacement of the button module 11 which may be required to couple or decouple further component parts of the dose setting and drive mechanism during the transition from a dose seting mode to a dose deliver mode. It is preferable, to limit the detrimental effect on dispense force, for the axial forces applied by the axial switch to the encoder ring 20 to be minimised. There is accommodation in the deflection of the switch contacts 23, 24 for over-travel of the switch chassis 22 after the switch contact 24 has contacted the second pad 27 of the PCB without the switch chassis 22 interfering with the PCB. This allows the switch to close in all tolerance conditions while maintaining a low reaction force on the proximal surface of the encoder ring 20.

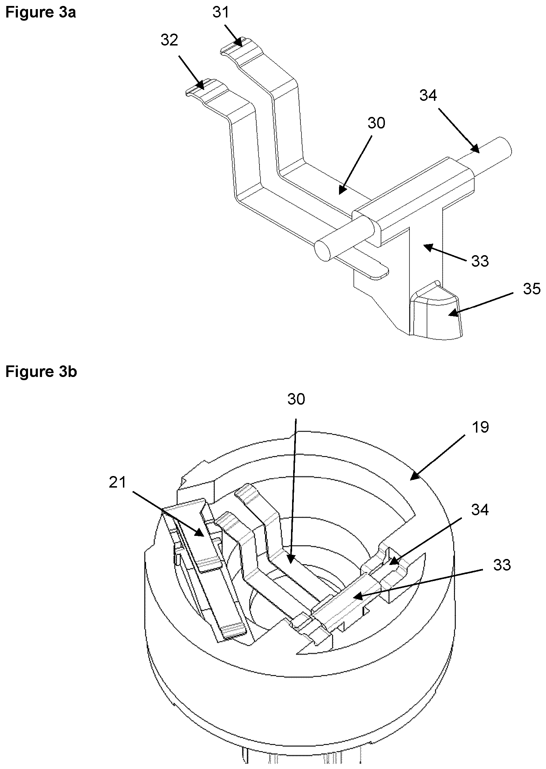

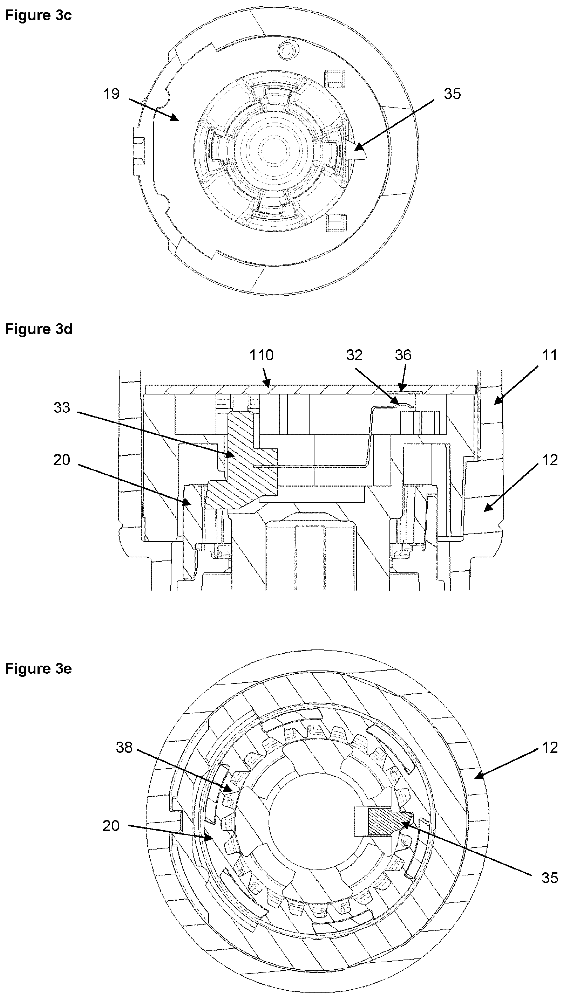

An additional or alternative electrical use detection unit 130 is described in the following with reference to Figures 3a to 3g depicting an embodiment with a rotational switch.

As shown in Figure 3a, a potential embodiment of the rotational switch comprises of a single rocker contact pressing 30 that is manufactured from stainless steel or beryllium copper etc. sheet or strip. This rocker contact pressing 30 has two contact arms, the tips of which, in the assembled orientation, are arranged with one more proximal than the other. The tips of the arms each form a respective switch contact 31, 32. The rocker contact pressing 30 is assembled onto a rocker 33 with e.g. two arms 34 for pivotably mounting the rocker 33 into the module chassis 19 as described above or a similar chassis component of the button module 11.

As shown in Figure 3b, the rocker sub-assembly is initially positioned into the module chassis 19 of the button module 11 sub-assembly. A tip or radially extending protrusion 35 of the rocker 33 component protrudes through a hole in the module chassis 19. in Figures 3a and 3c, the protrusion 35 has the shape of a tooth as will be explained below in more detail. The arms 34 of the rocker 33 form an axle which is clipped into pockets in the module chassis 19, such that radial motion of the protrusion 35 of the rocker 33 results in axial motion at the rocker switch contacts 31, 32.

When the PCB is assembled onto the module chassis 19 the rocker sub-assembly is compressed into the final assembly position. The PCB has two (further) exposed metallic pads on the bottom surface from which only one pad 36 is visible in Figure 3d. In the nominal tolerance condition the more proximal of the arms, i.e. the preload arm with switch contact 31, on the rocker is in contact with one of the exposed pads 37 and is connected electronically to the PCB. The compression of the preload arm provides a small pre-load force, which ensures the rocker tip or protrusion 35 is biased radially out of the module chassis 19. The other arm of the rocker 33, i.e. the switching arm with contact 32, is in clearance to the PCB so that it is in a first electrical state (for example electrically open circuit in the depicted embodment).

A radial ratchet profile 38 is moulded in the encoder ring 20 which forms part of the dial sleeve assembly. In the dialling state, when the button module 11 is assembled to the rest of the mechanism as shown in Figures 3f to 3g, the protrusion 35 of the rocker 33 sits in a root of a ratchet tooth of ratchet profile 38. The switching arm with contact 32 therefore remains in clearance to the PCB.

When the button module 11 is pressed it is displaced axially with respect to the dial sleeve assembly. The rocker protrusion 35 remains in the same root of a ratchet tooth during this initial axial travel. When dispensing begins, the dial sleeve assembly rotates relative to the button module 11 and the rocker protrusion 35 is pushed radially into the module chassis 19 each time a ratchet tooth on the encoder ring 20 passes the rocker protrusion 35. As shown in Figure 3g, the rocker sub-assembly rotates about the rocker axle 34, and the contact 32 of the switching arm moves proximally to close the clearance to the second contact pad 36 on the PCB. Once this clearance has been closed, the electrical state of the switch is changed (for example to electrically closed in the depicted embodiment).

It is possible to invert the arrangement so that the shape of the rocker protrusion 35 defines the cam profile and the ratchet teeth on the inner diameter of the encoder ring 20 become a series of cam followers. Using the rocker protrusion 35 as the driving profile reduces potential variation in the torque loss that may result from inconsistencies in the ratchet teeth profiles. In addition, the alternative arrangement may offer superior robustness since the width and therefore strength of the rocker protrusion 35 can be increased and wear will not be focussed on a single point on the rocker protrusion 35.

In an alternative, the profile of the encoder ratchet 38 can be optimised to minimise the peak torque loss as a result of deflecting the rocker protrusion 35 radially during dispense. As the rocker protrusion 35 is deflected radially inwards, the deflection induced in the preload and switching arms increases. Consequently, the radial reaction force of the rocker protrusion 35 acting on the encoder ring 20 increases as the rocker protrusion 35 is deflected radially inwards. The ramp profile of the ratchet teeth is configured to maximise the radial deflection induced in the rocker protrusion 35 when the reaction force from it is near a minimum (i.e. when the rocker protrusion 35 has not been significantly deflected). This is embodied as a ratchet ramp profile that varies from near radial at its maximum diameter to tangential or near tangential as it approaches its minimum diameter. In the embodiment described the encoder ring 20 creates a cam profile and the rocker protrusion 35 acts as a cam follower.

Although the time taken to initialise the encoder system is short, a delay between the start of dispensing ans the start of encoding a dispensed dose may have detrimental implications of on accuracy of encoding a dispensed dose. It is possible in very fast dispense events, that the system may dispense 1 or 2 units before the encoder system has had time to initialise, and therefore there could be an error in the reported dose. The system may use Gray code caching (e.g. as described in unpublished EP 20315066.9 and EP20315305.1) to compare the orientation of the dial sleeve assembly at the end of dose, with where it would be expected to be given the stored dial sleeve orientation at the end of the previous dose and the dispense volume recorded by the encoder system. The reported dose volume can then be retrospectively corrected. It is possible to detect and correct an error of up to 3U which is expected to be sufficient for all dispense cases.

Although described mainly with respect to a drug delivery device having a similar working principle as the device disclosed in EP 2 890 435, the electronic system is applicable to any other type of drug delivery device having component parts performing a relative axial and/or rotational movement in defined conditions or states.

### Reference Numerals

- 1: device
- 10: housing
- 11: button module
- 12: dial grip
- 13: dosage window
- 14: container/container receptacle
- 15: needle
- 16: inner needle cap
- 17: outer needle cap
- 18: cap
- 19: module chassis
- 20: encoder ring (dial sleeve assembly)
- 21: switch contact strip
- 22: switch chassis
- 23, 24: switch contact
- 25: protrusion (pin)
- 26, 27: contact pad
- 28: running face
- 30: switch contact pressing
- 31, 32: switch contact
- 33: rocker
- 34: arm (axle)
- 35: protrusion (tip)
- 36, 37: contact pad
- 38: ratchet profile
- 100: electronic system
- 110: electronic control unit (PCBA)
- 120: encoding and motion sensing unit
- 130: use detection unit
- 140: communication unit
- 150: electrical power supply

## Claims

1. An electronic system for a drug delivery device (1), the electronic system comprising:
- a dose setting and drive mechanism which is configured to perform a dose setting operation for setting a dose to be delivered by the drug delivery device and a dose delivery operation for delivering the set dose, the dose setting and drive mechanism comprising a first member (20),
- a button module (11) comprising an electronic control unit (110) connected to at least a rotary sensor (120), a communication unit (140) for communicating with another device and a use detection unit (130) with a rotational switch and an axial switch, wherein the electronic control unit (110) is configured to control an operation of the electronic system and has a distal surface facing towards the dose setting and drive mechanism,
wherein the button module (1) and the dose setting and drive mechanism are configured such that the first member (20) rotates relative to the button module (11) during the dose delivery operation but does not rotate relative to the button module (11) during the dose setting operation and that the button module (11) moves axially relative to the first member (20) during the transition from the dose setting operation to the dose delivery operation, or when the button module (11) is pressed in a 0U dialled condition,
**characterized in that** the switches comprise at least four contact pads (26, 27, 36, 37) at the distal surface of the electronic control unit (110) and at least two switch members each having at least two corresponding switch contacts (23, 24, 31, 32) defining a first electrical state, e.g. an electrically open circuit, if one of the switch contacts (24, 32) is not connected to a corresponding contact pad (27, 36) and a second electrical state, e.g. an electrically closed circuit, if both contact pads (26, 27, 36, 37) of the respective switch member are connected to the corresponding switch contacts (23, 24, 31, 32),
wherein rotatation of the first member (20) relative to the button module (11) generates a, preferably axial, movement component of the switch member of the rotational switch relative to the button module (11) and the first member (20), thereby switching between the first and second electrical states which in turn triggers activation of the rotary sensor (120),
wherein axial movement of the button module (11) relative to the first member (20) generates a, preferably axial, movement component of switch member of the axial switch relative to the button module (11) and the first member (20), thereby switching between the first and second electrical states which in turn activates the communication unit (140).

2. The electronic system according to claim 1, wherein the rotary sensor (120) comprises at least one of a light source with a corresponding optical sensor, an electrical sliding contact, a mechanical switching arrangement and/or a magnetic sensor.

3. The electronic system according to any one of claims 1 or 2, wherein each switch member comprises at least a first elastically deformable switch contact (23, 31) abutting or connected to one of the contact pads (26) and a second elastically deformable switch contact (24, 32) arranged axially offset to the first elastically deformable switch contact (23, 31) and spaced from one of the further contact pads (27, 36).

4. The electronic system according to any one of claims 1 to 3, wherein the axial switch is operable by a relative axial displacement of the button module (11) and the first member (20), which axial switch comprises a switch chassis (22) constraining a switch contact strip (21) comprising the elastically deformable switch contacts (23, 24).

5. The electronic system according to claim 4, wherein the switch contact strip (21) is substantially U-shaped with two legs each forming one of the elastically deformable switch contacts (23, 24), and wherein the switch contact strip (21) is arranged such that the legs are axially offset to each other.

6. The electronic system according to any one of claims 4 or 5, wherein the switch chassis (22) is interposed between a module chassis (19) of the button module (11) and the electronic control unit (110) with an axially extending protrusion (25) of the switch chassis (22) extending towards the first member (20), such that when the button module (11) moves axially towards the first member (20), one of the switch contacts (24) is brought into abutment with one of the contact pads (27).

7. The electronic system according to any one of the preceding claims, wherein the rotational switch is operable by a relative rotation of the button module (11) and the first member (20), which rotational switch comprises a rocker (33) constraining a switch contact pressing (30) comprising the elastically deformable switch contacts (31, 32), wherein the rocker (33) is pivotably guided in the button module (11).

8. The electronic system according to claim 7, wherein the switch contact pressing (30) comprises two contact arms, the tips of which are axially offset to each other and form elastically deformable switch contacts (31, 32).

9. The electronic system according to any one of claims 7 or 8, wherein the rocker (33) is interposed between a module chassis (19) of the button module (11) and the electronic control unit (110) with a radially extending protrusion (35) of the rocker (33) extending towards the first member (20), such that when the button module (11) rotates relative to the first member (20) a radial motion of the protrusion (35) of the rocker (33) results in an axial motion at the switch contacts (31, 32) and one of the switch contacts (32) is brought into abutment with one of the contact pads (36).

10. The electronic system according to any one of the preceding claims, wherein the first member is a dial sleeve assembly, e.g. a number sleeve, or a member (20) axially and/or rotationally locked thereto which first member is rotatable relative to a housing (10) of the dose setting and drive mechanism, e.g. along a helical path, at least in the dose setting operation and wherein the button module (11) is axially displaceable relative to the first member and rotationally constrained to the housing (10) at least in the dose delivery operation.

11. The electronic system according to claims 6 or 9 and 10, wherein the first member comprises an encoder ring (20) having a radial ratchet profile (38) for interaction with the radially extending protrusion (35) of the rocker (33) of the rotational switch and a proximally facing, ring-shaped running face (28) for interaction with the axially extending protrusion (25) of the switch chassis (22) of the axial switch.

12. The electronic system according to any one of the preceeding claims, wherein
- the rotary sensor (120) is in a sleeping mode in a first low power consumption state and is configured to be activated in a second high power consumption state, with the electronic system being configured such that it is switched from the first state into the second state by the electronic control unit in response to a signal of the rotational swich, thereby inducing the rotary sensor (120) to initiate a motion detection, and/or
- the communication unit (140) is in a sleeping mode in a first low power consumption state and is configured to be activated in a second high power consumption state, wherein, preferably, the communication unit (140) comprises a wireless communication interface for communicating with another device, with the electronic system being configured such that it is switched from the first state into the second state by the electronic control unit in response to a signal of the axial switch, thereby inducing the communication unit (140) to initiate a manual syncronsation and/or a pairing with another device.

13. The electronic system according to claim 12, wherein the electronic control unit (110) is adapted to switch the rotary sensor (120) into its low power consumption state in response to a signal that the axial switch is switched from its first electrical state, e.g. an electrically open circuit, into its second electrical state, e.g. an electrically closed circuit.

14. The electronic system according to claims 12 or 13, wherein the electronic control unit (110) is adapted to generate a failure indicating signal, e.g. transmitted by means of the communication unit (140), if a difference in rotational positon is detected by the rotary sensor (120) without detection of a state change of the rotational switch or if a rotational switch state change is received with no corresponding axial switch state change.

15. A drug delivery device comprising the electronic system according to any one of the preceeding claims and further comprising a container receptacle (14) which is permanently or releasably connected to the dose setting and drive mechanism and which is adapted to receive a container containing a medicament.

## Patentansprüche

1. Elektronisches System für eine Medikamenten-Verabreichungsvorrichtung (1), wobei das elektronische System Folgendes umfasst:
- einen Dosiseinstell- und Antriebsmechanismus, der dazu ausgelegt ist, einen Dosiseinstellvorgang zum Einstellen einer von der Medikamenten-Verabreichungsvorrichtung abzugebenden Dosis und einen Dosisabgabevorgang zum Abgeben der eingestellten Dosis durchzuführen, wobei der Dosiseinstell- und Antriebsmechanismus ein erstes Element (20) umfasst,
- ein Tastenmodul (11), das eine elektronische Steuereinheit (110), die mit mindestens einem Drehsensor (120) verbunden ist, eine Kommunikationseinheit (140) zum Kommunizieren mit einer anderen Vorrichtung und eine Verwendungsdetektionseinheit (130) mit einem Drehschalter und einem Axialschalter umfasst, wobei die elektronische Steuereinheit (110) dazu ausgelegt ist, einen Betrieb des elektronischen Systems zu steuern, und eine distale Fläche aufweist, die dem Dosiseinstell- und Antriebsmechanismus zugewandt ist,
wobei das Tastenmodul (1) und der Dosiseinstell- und Antriebsmechanismus so ausgelegt sind, dass sich das erste Element (20) während des Dosisabgabevorgangs relativ zu dem Tastenmodul (11) dreht, sich jedoch während des Dosiseinstellvorgangs nicht relativ zu dem Tastenmodul (11) dreht, und dass sich das Tastenmodul (11) während des Übergangs von dem Dosiseinstellvorgang zu dem Dosisabgabevorgang oder bei Drücken des Tastenmoduls (11) in einem angewählten 0U-Zustand relativ zu dem ersten Element (20) axial bewegt,
**dadurch gekennzeichnet, dass** die Schalter mindestens vier Kontaktflächen (26, 27, 36, 37) an der distalen Fläche der elektronischen Steuereinheit (110) und mindestens zwei Schaltelemente umfassen, die jeweils mindestens zwei entsprechende Schaltkontakte (23, 24, 31, 32) aufweisen, die einen ersten elektrischen Zustand, z. B. einen elektrisch offenen Stromkreis, wenn einer der Schaltkontakte (24, 32) nicht mit einer entsprechenden Kontaktfläche (27, 36) verbunden ist, und einen zweiten elektrischen Zustand, z. B. einen elektrisch geschlossenen Stromkreis, wenn beide Kontaktflächen (26, 27, 36, 37) des jeweiligen Schaltelements mit den entsprechenden Schaltkontakten (23, 24, 31, 32) verbunden sind, definieren,
wobei eine Drehung des ersten Elements (20) relativ zu dem Tastenmodul (11) eine, vorzugsweise axiale, Bewegungskomponente des Schaltelements des Drehschalters relativ zu dem Tastenmodul (11) und dem ersten Element (20) erzeugt, wodurch zwischen dem ersten und dem zweiten elektrischen Zustand umgeschaltet wird, was wiederum eine Aktivierung des Drehsensors (120) auslöst,
wobei eine axiale Bewegung des Tastenmoduls (11) relativ zu dem ersten Element (20) eine, vorzugsweise axiale, Bewegungskomponente des Schaltelements des Axialschalters relativ zu dem Tastenmodul (11) und dem ersten Element (20) erzeugt, wodurch zwischen dem ersten und dem zweiten elektrischen Zustand umgeschaltet wird, was wiederum die Kommunikationseinheit (140) aktiviert.

2. Elektronisches System nach Anspruch 1, wobei der Drehsensor (120) mindestens eines einer Lichtquelle mit einem entsprechenden optischen Sensor, eines elektrischen Gleitkontakts, einer mechanischen Schaltanordnung und/oder eines Magnetsensors umfasst.

3. Elektronisches System nach einem der Ansprüche 1 oder 2, wobei jedes Schaltelement mindestens einen ersten elastisch verformbaren Schaltkontakt (23, 31), der an einer der Kontaktflächen (26) anliegt oder damit verbunden ist, und einen zweiten elastisch verformbaren Schaltkontakt (24, 32), der axial von dem ersten elastisch verformbaren Schaltkontakt (23, 31) versetzt und von einer der weiteren Kontaktflächen (27, 36) beabstandet angeordnet ist, umfasst.

4. Elektronisches System nach einem der Ansprüche 1 bis 3, wobei der Axialschalter durch eine relative axiale Verschiebung des Tastenmoduls (11) und des ersten Elements (20) betätigbar ist, wobei der Axialschalter ein Schalterchassis (22) umfasst, das einen Schaltkontaktstreifen (21), der die elastisch verformbaren Schaltkontakte (23, 24) umfasst, zurückhält.

5. Elektronisches System nach Anspruch 4, wobei der Schaltkontaktstreifen (21) im Wesentlichen U-förmig mit zwei Schenkeln ist, die jeweils einen der elastisch verformbaren Schaltkontakte (23, 24) bilden, und wobei der Schaltkontaktstreifen (21) so angeordnet ist, dass die Schenkel axial voneinander versetzt sind.

6. Elektronisches System nach einem der Ansprüche 4 oder 5, wobei das Schalterchassis (22) zwischen einem Modulchassis (19) des Tastenmoduls (11) und der elektronischen Steuereinheit (110) angeordnet ist, wobei sich ein sich axial erstreckender Vorsprung (25) des Schalterchassis (22) so in Richtung des ersten Elements (20) erstreckt, dass, wenn sich das Tastenmodul (11) axial zu dem ersten Element (20) hin bewegt, einer der Schaltkontakte (24) in Anlagekontakt mit einer der Kontaktflächen (27) gebracht wird.

7. Elektronisches System nach einem der vorhergehenden Ansprüche, wobei der Drehschalter durch eine relative Drehung des Tastenmoduls (11) und des ersten Elements (20) betätigbar ist, wobei der Drehschalter eine Wippe (33) umfasst, die eine Schaltkontaktandrückvorrichtung (30), die die elastisch verformbaren Schaltkontakte (31, 32) umfasst, zurückhält, wobei die Wippe (33) schwenkbar in dem Tastenmodul (11) geführt ist.

8. Elektronisches System nach Anspruch 7, wobei die Schaltkontaktandrückvorrichtung (30) zwei Kontaktarme umfasst, deren Spitzen axial voneinander versetzt sind und elastisch verformbare Schaltkontakte (31, 32) bilden.

9. Elektronisches System nach einem der Ansprüche 7 oder 8, wobei die Wippe (33) zwischen einem Modulchassis (19) des Tastenmoduls (11) und der elektronischen Steuereinheit (110) angeordnet ist, wobei sich ein sich radial erstreckender Vorsprung (35) der Wippe (33) so zu dem ersten Element (20) hin erstreckt, dass, wenn sich das Tastenmodul (11) relativ zu dem ersten Element (20) dreht, eine radiale Bewegung des Vorsprungs (35) der Wippe (33) zu einer axialen Bewegung an den Schaltkontakten (31, 32) führt und einer der Schaltkontakte (32) in Anlagekontakt mit einer der Kontaktflächen (36) gebracht wird.

10. Elektronisches System nach einem der vorhergehenden Ansprüche, wobei das erste Element eine Wahlhülsenanordnung, z. B. eine Zahlenhülse, oder ein Element (20) ist, das axial und/oder drehfest daran verriegelt ist, wobei das erste Element zumindest bei dem Dosiseinstellvorgang relativ zu einem Gehäuse (10) des Dosiseinstell- und Antriebsmechanismus, z. B. entlang einer spiralförmigen Bahn, drehbar ist und wobei das Tastenmodul (11) zumindest bei dem Dosisabgabevorgang relativ zu dem ersten Element axial verschiebbar ist und drehfest an dem Gehäuse (10) zurückgehalten ist.

11. Elektronisches System nach Anspruch 6 oder 9 und 10, wobei das erste Element einen Encoderring (20) mit einem radialen Klinkenprofil (38) zum Zusammenwirken mit dem sich radial erstreckenden Vorsprung (35) der Wippe (33) des Drehschalters und einer proximal ausgerichteten, ringförmigen Lauffläche (28) zum Zusammenwirken mit dem sich axial erstreckenden Vorsprung (25) des Schalterchassis (22) des Axialschalters umfasst.

12. Elektronisches System nach einem der vorhergehenden Ansprüche, wobei
- sich der Drehsensor (120) in einem Ruhemodus in einem ersten Zustand mit niedrigem Stromverbrauch befindet und dazu ausgelegt ist, in einen zweiten Zustand mit hohem Stromverbrauch aktiviert zu werden, wobei das elektronische System so ausgelegt ist, dass es als Reaktion auf ein Signal des Drehschalters durch die elektronische Steuereinheit von dem ersten Zustand in den zweiten Zustand umgeschaltet wird, wodurch der Drehsensor (120) veranlasst wird, eine Bewegungsdetektion zu initiieren, und/oder
- sich die Kommunikationseinheit (140) in einem Ruhemodus in einem ersten Zustand mit niedrigem Stromverbrauch befindet und dazu ausgelegt ist, in einen zweiten Zustand mit hohem Stromverbrauch aktiviert zu werden, wobei vorzugsweise die Kommunikationseinheit (140) eine drahtlose Kommunikationsschnittstelle zum Kommunizieren mit einer anderen Vorrichtung umfasst, wobei das elektronische System so ausgelegt ist, dass es als Reaktion auf ein Signal des Axialschalters durch die elektronische Steuereinheit von dem ersten Zustand in den zweiten Zustand umgeschaltet wird, wodurch die Kommunikationseinheit (140) veranlasst wird, eine manuelle Synchronisation und/oder eine Kopplung mit einer anderen Vorrichtung zu initiieren.

13. Elektronisches System nach Anspruch 12, wobei die elektronische Steuereinheit (110) dazu geeignet ist, als Reaktion auf ein Signal, dass der Axialschalter von seinem ersten elektrischen Zustand, z. B. einem elektrisch offenen Stromkreis, in seinen zweiten elektrischen Zustand, z. B. einen elektrisch geschlossenen Stromkreis, geschaltet wird, den Drehsensor (120) in seinen Zustand mit niedrigem Stromverbrauch umzuschalten.

14. Elektronisches System nach Anspruch 12 oder 13, wobei die elektronische Steuereinheit (110) dazu geeignet ist, ein Fehleranzeigesignal zu erzeugen, das z. B. mittels der Kommunikationseinheit (140) übertragen wird, wenn eine Differenz der Drehposition durch den Drehsensor (120) ohne Detektion einer Zustandsänderung des Drehschalters detektiert wird oder wenn eine Drehschalterzustandsänderung ohne eine entsprechende Axialschalterzustandsänderung empfangen wird.

15. Medikamenten-Verabreichungsvorrichtung mit dem elektronischen System nach einem der vorhergehenden Ansprüche und ferner umfassend eine Behälteraufnahme (14), die dauerhaft oder lösbar mit dem Dosiseinstell- und Antriebsmechanismus verbunden ist und die dazu geeignet ist, einen ein Arzneimittel enthaltenden Behälter aufzunehmen.

## Revendications

1. Système électronique pour dispositif d'administration de médicament (1), le système électronique comprenant :
- un mécanisme de réglage et d'entraînement de dose qui est configuré pour effectuer une opération de réglage de dose pour régler une dose à administrer par le dispositif d'administration de médicament et une opération d'administration de dose pour administrer la dose réglée, le mécanisme de réglage et d'entraînement de dose comprenant un premier élément (20),
- un module de bouton (11) comprenant une unité de commande électronique (110) connectée à au moins un capteur rotatif (120), une unité de communication (140) pour communiquer avec un autre dispositif et une unité de détection d'utilisation (130) avec un commutateur rotatif et un commutateur axial, l'unité de commande électronique (110) étant configurée pour commander un fonctionnement du système électronique et ayant une surface distale tournée vers le mécanisme de réglage et d'entraînement de dose,
le module de bouton (1) et le mécanisme de réglage et d'entraînement de dose étant configurés de telle sorte que le premier élément (20) tourne par rapport au module de bouton (11) pendant l'opération d'administration de dose, mais ne tourne pas par rapport au module de bouton (11) pendant l'opération de réglage de dose et que le module de bouton (11) se déplace axialement par rapport au premier élément (20) pendant le passage entre l'opération de réglage de dose et l'opération d'administration de dose, ou lorsque le module de bouton (11) est enfoncé dans un état réglé sur 0U,
**caractérisé en ce que** les commutateurs comprennent au moins quatre plages de contact (26, 27, 36, 37) au niveau de la surface distale de l'unité de commande électronique (110) et au moins deux éléments de commutation présentant chacun au moins deux contacts de commutation (23, 24, 31, 32) correspondants définissant un premier état électrique, par exemple un circuit électriquement ouvert, si un des contacts de commutation (24, 32) n'est pas connecté à une plage de contact correspondante (27, 36) et un second état électrique, par exemple un circuit fermé électriquement, si les deux plages de contact (26, 27, 36, 37) de l'élément de commutation respectif sont connectées aux contacts de commutation correspondants (23, 24, 31, 32), la rotation du premier élément (20) par rapport au module de bouton (11) générant une composante de déplacement, de préférence axiale, de l'élément de commutation du commutateur rotatif par rapport au module de bouton (11) et au premier élément (20), commutant ainsi entre les premier et second états électriques qui à son tour déclenche l'activation du capteur rotatif (120),
le mouvement axial du module de bouton (11) par rapport au premier élément (20) générant une composante de mouvement, de préférence axiale, de l'élément de commutation du commutateur axial par rapport au module de bouton (11) et au premier élément (20), commutant ainsi entre les premier et second états électriques qui à son tour active l'unité de communication (140).

2. Système électronique selon la revendication 1, le capteur rotatif (120) comprenant au moins l'un d'une source de lumière avec un capteur optique correspondant, un contact électrique glissant, un agencement de commutation mécanique et/ou un capteur magnétique.

3. Système électronique selon l'une quelconque des revendications 1 ou 2, chaque élément de commutation comprenant au moins un premier contact de commutation élastiquement déformable (23, 31) butant contre ou connecté à l'une des plages de contact (26) et un second contact de commutation élastiquement déformable (24, 32) agencé de manière décalée axialement par rapport au premier contact de commutation élastiquement déformable (23, 31) et espacé de l'une des autres plages de contact (27, 36).

4. Système électronique selon l'une quelconque des revendications 1 à 3, le commutateur axial pouvant être actionné par un déplacement axial relatif du module de bouton (11) et du premier élément (20), lequel commutateur axial comprenant un châssis de commutateur (22) qui contraint une bande de contact de commutation (21) comprenant les contacts de commutation élastiquement déformables (23, 24).

5. Système électronique selon la revendication 4, la bande de contact de commutation (21) étant sensiblement en forme de U avec deux branches formant chacune l'un des contacts de commutation élastiquement déformables (23, 24), et la bande de contact de commutation (21) étant agencée de telle sorte que les branches sont axialement décalées l'une par rapport à l'autre.

6. Système électronique selon l'une quelconque des revendications 4 ou 5, le châssis de commutateur (22) étant interposé entre un châssis de module (19) du module de bouton (11) et l'unité de commande électronique (110) avec une saillie (25) s'étendant axialement du châssis de commutateur (22) s'étendant vers le premier élément (20), de sorte que lorsque le module de bouton (11) se déplace axialement vers le premier élément (20), l'un des contacts de commutation (24) est mis en butée contre l'une des plages de contact (27).

7. Système électronique selon l'une quelconque des revendications précédentes, le commutateur rotatif pouvant être actionné par une rotation par rapport au module de bouton (11) et du premier élément (20), lequel commutateur rotatif comprenant une bascule (33) qui contraint un poussoir de contact de commutation (30) comprenant les contacts de commutation élastiquement déformables (31, 32), la bascule (33) étant guidée de manière pivotante dans le module de bouton (11).

8. Système électronique selon la revendication 7, le poussoir de contact de commutation (30) comprenant deux bras de contact dont les pointes sont décalées axialement l'une par rapport à l'autre et forment des contacts de commutation élastiquement déformables (31, 32).

9. Système électronique selon l'une quelconque des revendications 7 ou 8, la bascule (33) étant interposée entre un châssis de module (19) du module de bouton (11) et l'unité de commande électronique (110) avec une saillie (35) s'étendant radialement de la bascule (33) s'étendant vers le premier élément (20), de sorte que lorsque le module de bouton (11) tourne par rapport au premier élément (20), un mouvement radial de la saillie (35) de la bascule (33) entraîne un mouvement axial au niveau des contacts de commutation (31, 32) et que l'un des contacts de commutation (32) est mis en butée contre l'une des plages de contact (36).

10. Système électronique selon l'une quelconque des revendications précédentes, le premier élément étant un assemblage de manchon de cadran, par exemple un manchon numéroté, ou un élément (20) verrouillé axialement et/ou en rotation sur celui-ci, le premier élément pouvant tourner par rapport à un logement (10) du mécanisme de réglage et d'entraînement de dose, par exemple le long d'un trajet hélicoïdal, au moins lors de l'opération de réglage de dose, et le module de bouton (11) pouvant se déplacer axialement par rapport au premier élément et être contraint en rotation sur le logement (10) au moins lors de l'opération d'administration de dose.

11. Système électronique selon les revendications 6 ou 9 et 10, le premier élément comprenant une bague codeuse (20) ayant un profil de cliquet radial (38) pour interaction avec la saillie s'étendant radialement (35) de la bascule (33) du commutateur rotatif et une face de roulement en forme d'anneau orientée proximalement (28) pour interaction avec la saillie s'étendant axialement (25) du châssis de commutateur (22) du commutateur axial.

12. Système électronique selon l'une quelconque des revendications précédentes,
- le capteur rotatif (120) étant dans un mode veille dans un premier état de faible consommation d'énergie et étant configuré pour être activé dans un second état de forte consommation d'énergie, le système électronique étant configuré pour être commuté du premier état dans le second état par l'unité de commande électronique en réponse à un signal du commutateur rotatif, induisant ainsi le capteur rotatif (120) à initier une détection de mouvement, et/ou
- l'unité de communication (140) étant dans un mode veille dans un premier état de faible consommation d'énergie et étant configurée pour être activée dans un second état de forte consommation d'énergie, de préférence, l'unité de communication (140) comprenant une interface de communication sans fil pour communiquer avec un autre dispositif, le système électronique étant configuré pour être basculé du premier état dans le second état par l'unité de commande électronique en réponse à un signal du commutateur axial, induisant ainsi l'unité de communication (140) à initier une synchronisation manuelle et/ou un appariement avec un autre dispositif.

13. Système électronique selon la revendication 12, l'unité de commande électronique (110) étant adaptée pour commuter le capteur rotatif (120) dans son état de faible consommation d'énergie en réponse à un signal selon lequel le commutateur axial est commuté de son premier état électrique, par exemple un circuit ouvert électriquement, à son second état électrique, par exemple un circuit fermé électriquement.

14. Système électronique selon la revendication 12 ou 13, l'unité de commande électronique (110) étant adaptée pour générer un signal indiquant une défaillance, par exemple transmis au moyen de l'unité de communication (140), si une différence de position de rotation est détectée par le capteur rotatif (120) sans détection d'un changement d'état du commutateur rotatif ou si un changement d'état du commutateur rotatif est reçu sans changement d'état du commutateur axial correspondant.

15. Dispositif d'administration de médicament comprenant le système électronique selon l'une quelconque des revendications précédentes, et comprenant en outre un réceptacle de récipient (14) qui est connecté de manière permanente ou amovible au mécanisme de réglage et d'entraînement de dose et qui est adapté pour recevoir un récipient contenant un médicament.
